# EUROPÄISCHE PATENTSCHRIFT

(11) **EP 3 830 567 B1**
(45) Veröffentlichungstag und Bekanntmachung des Hinweises auf die Patenterteilung: **27.08.2025**
(21) Anmeldenummer: 19752651.0
(22) Anmeldetag: 26.07.2019
(51) Int. Cl.: G01N 31/22, C07D 487/22, G01N 21/78, G01N 33/00, G01N 33/12, G01N 33/497, G01N 21/77

(54) **DOSIMETER-MATERIAL FÜR AMMONIAK UND/ODER AMINE, DESSEN HERSTELLUNG UND VERWENDUNG**
DOSIMETER MATERIAL FOR AMMONIA AND/OR AMINES, ITS FABRICATION AND USE
MATÉRIAU DOSIMÈTRE POUR AMMONIAC ET/OU AMINES, SA FABRICATION ET SON UTILISATION

(30) Priorität: 27.07.2018 DE 102018118211
(43) Veröffentlichungstag der Anmeldung: 09.06.2021
(73) Patentinhaber: Universität zu Lübeck, 23562 Lübeck (DE); Porphyrin-Laboratories GmbH, 23684 Scharbeutz (DE)
(72) Erfinder: RAHMANZADEH, Ramtin, 23566 Lübeck (DE); HÜTTMANN, Gereon, 23564 Lübeck (DE); SCHELL, Christian, 23617 Stockelsdorf (DE)
(74) Vertreter: Moré, Solveig Helga
(86) Internationale Anmeldenummer: PCT/EP2019/070154
(87) Internationale Veröffentlichungsnummer: WO 2020/021053

(56) Entgegenhaltungen:
- US-B2- 7 772 215
- HIROSHI SEGAWA ET AL: "Photoinduced axial bond exchange reaction of dichlorophosphorus(V)tetraphenylporphyrin chloride: photochemical activation of central three-centre four-electron bond through the [sigma]-[pi] interaction", JOURNAL OF THE CHEMICAL SOCIETY, CHEMICAL COMMUNICATIONS, no. 15, 1 January 1992 (1992-01-01), pages 1066 - 1067, XP055652564, ISSN: 0022-4936, DOI: 10.1039/C39920001066
- JIN MATSUMOTO ET AL: "Water-Solubilization of P(V) and Sb(V) Porphyrins and Their Photobiological Application", INTERNATIONAL JOURNAL OF PHOTOENERGY, vol. 2015, 8 January 2015 (2015-01-08), pages 1 - 12, XP055652700, ISSN: 1110-662X, DOI: 10.1155/2015/148964

## Beschreibung

Die Erfindung betrifft ein Dosimeter-Material für Ammoniak und/oder Amine, den verwendeten Indikator, sowie Verfahren zu deren Herstellung und Verwendung, insbesondere zur Qualitätskontrolle bei Lebensmitteln.

In der Lebensmittelindustrie gibt es einen großen Bedarf, die Haltbarkeit und Frische von Lebensmitteln routinemäßig und nicht-invasiv zu bestimmen. Beim Abbau von biologischem Gewebe, so z.B. beim Verderb von tierischen Lebensmitteln werden Proteinabbauprodukte wie Amine in gasförmiger Phase in die Umgebung abgegeben. Über die Detektion dieser lässt sich die Frische von Lebensmitteln bestimmen und entsprechend eine eintretende Veränderung der Haltbarkeit (Verderb, Reife) indizieren. Bisher können Amine nur mit einer aufwendigen Laboranalytik nachgewiesen werden.

Bei der Analytik von Aminen in der Lebensmittelchemie/Qualitätskontrolle/ Lebensmittelüberwachung handelt es sich in der Regel um komplexe Verfahren mit hohem apparativen Aufwand, z.B. Gaschromatographie in Kombination mit Massenspektroskopie. Solche Techniken erfordern auch eine aufwendige und zeitintensive Probenvorbereitung. Des Weiteren ist für die Bedienung der komplexen Geräte geschultes Personal erforderlich. Diese insgesamt kostenaufwendige Bestimmung des Lebensmittelzustandes führt dazu, dass in der Produktion von Lebensmitteln eine überwachende Lebensmittelkontrolle nur stichprobenartig erfolgen kann. Sowohl in der Umweltanalytik als auch in der Medizinanalytik stellen sich ähnliche Problematiken dar. Insbesondere in der lebensmittelverarbeitenden Industrie wird dringend nach zeit- und kostengünstigen Verfahren gesucht, bei denen eine aussagekräftige Kontrolle der Haltbarkeit des verpackten Produkts bei jeder einzelnen Verpackung erfolgen kann.

Die allgemeine Idee der Qualitätskontrolle in der Gasphase bei verpackten Lebensmitteln ist aus dem Stand der Technik bekannt. Beispielsweise wird in der EP 0449798 A2 ein Verfahren zur Qualitätskontrolle von verpackten, organischen Stoffen vorgeschlagen, bei dem der organische Stoff zusammen mit einem optischen Sensorelement eingeschlossen und so mit der Gasphase zwischen organischem Stoff und Verpackung in Kontakt gebracht wird, so dass eine auf Zersetzung des organischen Stoffes beruhende Änderung der Zusammensetzung der Gasphase zu einer Änderung der Farbe des Sensorelementes führt, die visuell detektiert werden kann. Dieses Verfahren wird u. A. auch für die Detektion von Ammoniak oder Aminen vorgeschlagen. Eine spezifischere Variante hiervon ist die Verwendung von Porphyrinen, in Kombination mit einer Folie, als optischer Sensor für Amine. Auch dies ist aus dem Stand der Technik bekannt. Beispielsweise beschreibt die Gebrauchsmusterschrift DE 212010000225 U1 ein Verpackungsmaterial zur Bestimmung der Frische von Lebensmitteln, das aus einem Sensormaterial und einer Folie besteht, wobei die Detektion von bei der Zersetzung von Fisch bzw. Fleisch freigesetzten Ammoniak und Aminen mittels Porphyrinen erfolgen kann.

Verschiedene maßgeschneiderte Zink-(II)- und Chrom-(III)-metalloporphyrine wurden als Chromophore für den kolorimetrischen Nachweis von Aminen verwendet (Heier, P.C. (2014) Novel metallo-porphyrin based colourimetric amine sensors and their processing via plasma enhanced chemical vapour deposition at atmospheric pressure synthesis, characterisation and mechanistic studies. Dissertation, Universität Mainz). Für das Zink-(II)-Metalloporphyrin ist eine Verschiebung der Soret-Bande von 420 nm nach 433 nm und für das Chrom-(III)-Metalloporphyrin ist eine Verschiebung der Soret-Bande von 435 nm nach 452 nm zu beobachten. Ungünstig für die Verwendung als Aminsensor sind die geringen Absorptionsänderungen der eingesetzten Porphyrine. Ferner gelten Chrom-Porphyrine als gesundheitlich bedenklich, und somit ist deren Einsatz im Zusammenhang mit Lebensmitteln ist als kritisch anzusehen.

Im Stand der Technik sind Phosphorporphyrine mit der Formel Porphyrin-P(V)Cl₃ bekannt, welche durch Substitution der zwei Phosphor-gebundenen Chlor-Liganden durch Alkoxygruppen aktiviert wurden (Segawa et al., 1992. Journal of the Chemical Society, Chemical Communications 15, S. 1066-1067). Matsumoto et al., 2015, International Journal of Photoenergy, S. 1-12, offenbart verschiedene Phosphorporphyrine mit unterschiedlichen axialen Phosphorliganden für die photodynamische Therapie und die photodynamische Inaktivierung.

Aufgabe der Erfindung ist es, ein Dosimeter-Material zur Verfügung zu stellen, das auf vorbestimmte Konzentrationen von Ammoniak und/oder Aminen ohne Querempfindlichkeiten zu anderen Stoffen, insbesondere zu Wasser, eingestellt werden kann, das gesundheitlich unbedenklich ist, und das visuell, photometrisch und/oder fluorimetrisch ausgewertet werden kann.

Die Aufgabe wird durch den Gegenstand der Ansprüche gelöst, insbesondere durch ein Dosimeter-Material mit den Merkmalen des Anspruchs 4.

Gegenstand der Erfindung ist als Zwischenprodukt in der Herstellung des erfindungsgemäßen Dosimeter-Materials auch der Indikator nach Anspruch 1. Die Unteransprüche geben vorteilhafte Ausgestaltungen an.

Das erfindungsgemäße Dosimeter-Material für Ammoniak und/oder Amine umfasst einen Indikator, der in Anwesenheit von Ammoniak und/oder Aminen einen irreversiblen Farbwechsel erfährt, und eine für Ammoniak und/oder Amine permeable Immobilisierungsmatrix für den Indikator, wobei die Immobilisierungsmatrix wasserundurchlässig ist.

Der erfindungsgemäße Indikator umfasst ein durch kovalente Bindung an eine Silanolgruppe (auch als Silinolgruppe bezeichnet) aktiviertes Phosphorporphyrin mit der Formel Porphyrin-P(V)X₃, wobei X Cl oder Br ist, wobei eine Aktivierung des Porphyrin-P(V)X₃ durch eine kovalente Bindung durch die Reaktion nur eines der Halogen-Liganden des Porphyrin-P(V)X₃ mit der Silanolgruppe, bevorzugt von Kieselgel, erfolgt. Die Silanolgruppe ist Teil eines Stoffes, der eine Vielzahl von Silanolgruppen aufweist und eine hohe Oberfläche hat. Zum Beispiel umfasst der Indikator ein durch kovalente Bindung an eine Silanolgruppe von einem Kieselgel umfassenden Stoff aktiviertes Phosphorporphyrin mit der Formel Porphyrin-P(V)X₃, wobei X Cl oder Br ist. Bevorzugt umfasst der Indikator ein durch kovalente Bindung an eine Silanolgruppe von Kieselgel aktiviertes Phosphorporphyrin mit der Formel Porphyrin-P(V)X₃, wobei X Cl oder Br ist.

Bei Phosphorporphyrinen des Typs Porphyrin-P(V)X₃, wobei X insbesondere die Halogene Cl oder Br umfasst, sind zwei Halogenliganden axial am Phosphor gebunden, und ein Halogenidgegenion ist für den Ladungsausgleich des Komplexes verantwortlich.

Der erfindungsgemäße Indikator kann insbesondere Dibromo-phosphor-(V)-tetraphenylporphyrin-bromid (TPP-P(V)Br₃), Dichloro-phosphor-(V)-tetratolylporphyrin-chlorids (TTP-P(V)Cl₃), Dichloro-phosphor-(V)-2,3,7,8,12,13,17,18-octaethylporphyrin-chlorid (OEP-P(V)Cl₃) oder bevorzugt Dichloro-phosphor-(V)-tetraphenylporphyrin-chlorid (TPP-P(V)Cl₃) umfassen.

Ein einen erfindungsgemäßen Indikator umfassendes Dosimeter-Material ist besonders geeignet, um mit Ammoniak und/oder Aminen in der Gasphase zu reagieren.

Das erfindungsgemäße Dosimeter-Material ist ferner dadurch gekennzeichnet, dass eine Aktivierung des Porphyrin-P(V)X₃ durch kovalente Bindung eines der Halogen-Liganden an eine Silanolgruppe oberflächenreicher Stoffe, insbesondere bei Kieselgel, stattfindet.

Bevorzugt wird erfindungsgemäß ein Dosimeter-Material für Ammoniak und/oder Amine, bei dem der Indikator durch kovalente Bindung an Kieselgel aktiviertes Dichloro-phosphor-(V)-tetraphenylporphyrin-chlorid (TPP-P(V)Cl₃) umfasst.

Die Erfinder haben gezeigt, dass durch kovalente Bindung an Kieselgel aktiviertes TPP-P(V)Cl₃ überraschend irreversibel mit Ammoniak und/oder Aminen reagiert. Diese Reaktion führt zu einem irreversiblen Farbwechsel des TPP-P(V)Cl₃-Indikators. Dieses Verhalten ist allgemein bei der Reaktion von Phosphorporphyrin-Indikatoren mit der Formel Porphyrin-P(V)X₃ mit Silanolgruppen gegeben. Die Reaktion des Indikators mit Wasser verursacht ebenfalls einen Farbwechsel des Indikators. Da der Indikator sehr empfindlich auf Feuchtigkeitsspuren in der Gasphase reagiert, ist für den selektiven Nachweis von Ammoniak und/oder Aminen notwendig, eine mögliche Reaktion des Indikators mit Wasser zu vermeiden. Erfindungsgemäß wird dafür der Indikator in eine wasserundurchlässige Immobilisierungsmatrix eingebracht.

Die erfindungsgemäßen Phosphorporphyrin-Indikatoren, so auch der TPP-P(V)Cl₃-Indikator, reagieren mit Ammoniak und/oder mit Aminen im Stoffmengenverhältnis 1:1. Da diese Reaktion irreversibel verläuft, kann der irreversible Farbwechsel integrierend als vorhandene Dosis an Ammoniak und/oder Aminen über die Zeit in der analysierten Probe, insbesondere in einem Gasgemisch, interpretiert werden. Die erfindungsgemäße Kombination von Phosphorporphyrin-Indikator und wasserundurchlässiger Immobilisierungsmatrix kann somit als wasserunempfindliches Dosimeter für Ammoniak und/oder Aminen verwendet werden.

Als Farbwechsel ist erfindungsgemäß bevorzugt ein mit dem bloßen Auge qualitativ auswertbarer Wechsel von grün nach rot und zusätzlich eine deutliche Wellenlängenverschiebung innerhalb der Absorptions- und Fluoreszenzspektren - ggf. unter Änderung der Spektrenform - des Indikatormaterials gemeint, welche messtechnisch (evtl. automatisiert) quantitativ erfasst und ausgewertet werden kann.

Damit die zu untersuchenden Ammoniak und/oder Amine enthaltenden Proben optimal mit dem Phosphorporphyrin-Indikator, insbesondere mit dem TPP-P(V)Cl₃-Indikator, im Dosimeter-Material reagieren können, liegt das Dosimeter-Material z.B. als Granulat und/oder Folie, bevorzugt als Folie vor.

Das Dosimeter-Material ist ferner bevorzugt dadurch gekennzeichnet, dass die Immobilisierungsmatrix für Wasser undurchlässige und für Ammoniak und/oder Amine durchlässige Polymere umfasst, insbesondere Polystyrol und/oder bevorzugt Low-Density Polyethylen.

Das Verfahren zur Herstellung des Phosphorporphyrin-Indikators umfasst eine Aktivierung des Porphyrin-P(V)X₃ durch kovalente Bindung durch eine Reaktion nur eines der Halogen-Liganden des Porphyrin-P(V)X₃ mit einer Silanolgruppe des oberflächenreichen Stoffes, bevorzugt von Kieselgel. Diese Reaktion erfolgt bei erhöhter Temperatur, bevorzugt zwischen 80 und 140 °C, bevorzugt bei 120 °C, und bevorzugt für 8 bis 30 Stunden.

Ein bevorzugtes Verfahren zur Herstellung des TPP-P(V)Cl₃-Indikators umfasst eine Aktivierung des TPP-P(V)Cl₃ durch kovalente Bindung an Kieselgel durch die Reaktion eines der Chlor-Liganden des TPP-P(V)Cl₃ mit einer Silanolgruppe des Kieselgels.

Das Verfahren zur Herstellung des Dosimeter-Materials umfasst bevorzugt das Vermischen des Indikators mit der Immobilisierungsmatrix, wobei das Verfahren bevorzugt unter Ausschluss von Wasser durchgeführt wird, und das Gemisch aus Indikator und Immobilisierungsmatrix bevorzugt als Granulat und/oder Folie vorliegen kann.

Das erfindungsgemäße Dosimeter-Material kann zur Detektion von Ammoniak und/oder Aminen verwendet werden, wobei der Farbwechsel des Indikators bevorzugt visuell erfasst werden kann. Anwendungsgebiete dafür sind z.B. die Qualitätskontrolle von Lebensmitteln, medizinische Anwendungen wie die Atemgasanalyse oder Wundheilverbänden, oder die Umweltanalytik.

Weitere Vorteile, Merkmale und Anwendungsmöglichkeiten der vorliegenden Erfindung ergeben sich aus der nachfolgenden Beschreibung in Zusammenhang mit den Figuren. Es zeigen:
- Fig. 1: UV/VIS-Spektrum von Dichloro-phosphor-(V)-tetraphenylporphyrin-chlorid (TPP-P(V)Cl₃) in DCM.
- Fig. 2: Synthese von Tetraphenylporphyrin (TPP).
- Fig. 3: Phosphorylierung von TPP.
- Fig. 4: Bindung von TPP-P(V)Cl₃ an eine Silanolgruppe auf der Oberfläche von Kieselgel.
- Fig. 5: Reaktion eines Amins mit dem aktivierten Chlor-Ligand des TPP-P(V)Cl₃.
- Fig. 6: Farbwechsel des Indikatorpulvers: grün in Abwesenheit von Aminen (links) und rot nach Reaktion mit Aminen (rechts).
- Fig. 7: Farbwechsel des Dosimeter-Materialgranulats: grün in Abwesenheit von Aminen (links) und rot nach Reaktion mit Aminen (rechts).
- Fig. 8: Absorptions- (A) und Fluoreszenzspektren (B) des Indikators vor (grün) und nach (rot) der Reaktion mit Aminen.
- Fig. 9: Änderung der Fluoreszenzlebenszeit des Indikators: (A) grün, (B) rot.

Der Ausgangsstoff zur Herstellung des erfindungsgemäßen Indikators für Ammoniak und/oder Amine ist ein Phosphorporphyrin mit der allgemeinen Formel Porphyrin-P(V)X₃, beispielsweise Dichloro-phosphor-(V)-tetraphenylporphyrin-chlorid (TPP-P(V)Cl₃). Dieses ist ein mit Phenylresten in meso-Position substituiertes Porphyrin. In dem aromatischen Ringsystem ist fünfwertiger Phosphor koordinativ gebunden, der zwei axiale Chlor-Liganden enthält. Ladungsausgleich erfolgt über ein Chlorid-Gegenion.

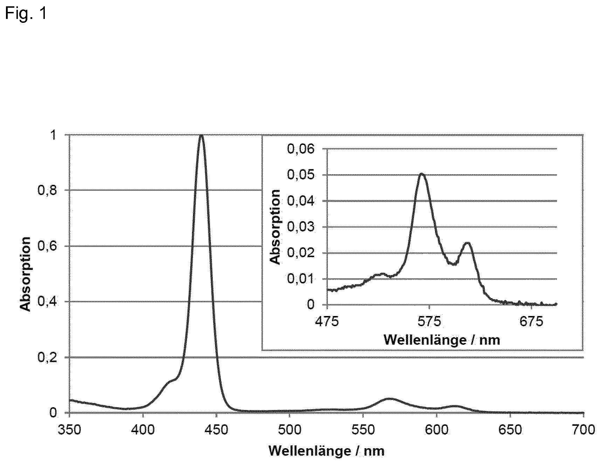

Phosphorporphyrine haben, wie auch andere Porphyrinkomplexe mit Elementen der fünften Hauptgruppe (Arsen, Antimon und Bismut), die Besonderheit, dass sie in zwei unterschiedlichen Oxidationsstufen vorkommen können. Die eher instabile Stufe +III, welche auch hypervalent genannt wird, und die stabile Oxidationsstufe +V. Diese unterscheiden sich unter anderem in ihrem Absorptionsspektrum. Phosphor-(V)-Porphyrine zeigen ein für Porphyrine typisches UV/VIS-Spektrum mit einer Soret-Bande und zwei Q-Banden (Fig. 1).

Phosphor-(V)-Porphyrine tragen zusätzlich zur koordinativen Bindung zu den Stickstoffatomen des Porphyrinrings und zu den beiden axialen Liganden (Halogenid, z.B. Chlorid) eine positive Ladung, die durch ein Halogenid-Anion, z.B. ein Chlorid-Anion, kompensiert wird. Die axialen Halogenid-Liganden können durch geeignete Nukleophile substituiert werden. Bekannt ist z.B. der Austausch gegen Halogen-, Hydroxy-, Alkoxy-, oder Aryloxy-Gruppen. Allerdings ist aus der Literatur bekannt, dass in Lösung immer nur beide Liganden substituiert werden. Erfindungsgemäß wird hier im Gegensatz dazu nur einer der Halogenid-Liganden des Porphyrin-P(V)X₃, insbesondere nur einer der Chlor-Liganden des TPP-P(V)Cl₃, aktiviert.

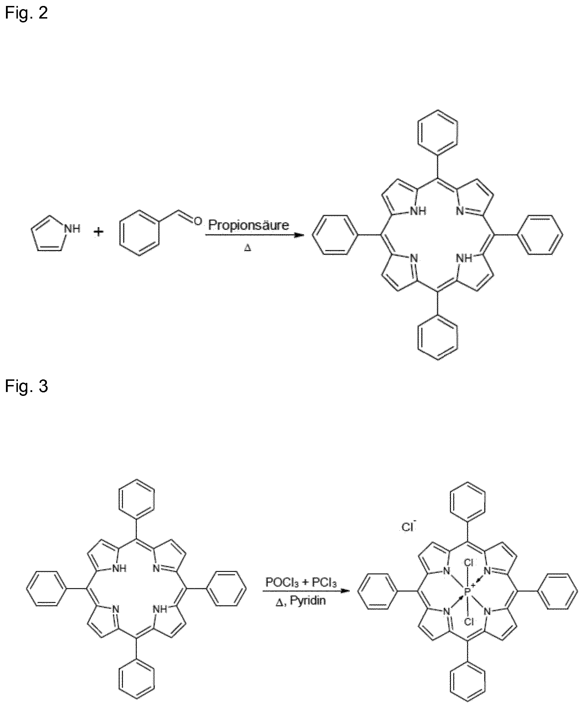

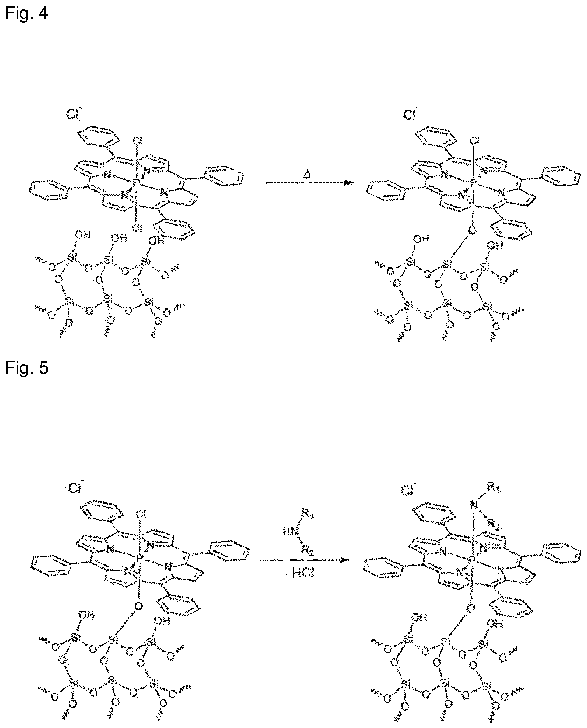

Das bevorzugte erfindungsgemäße Verfahren zur Herstellung des TPP-P(V)Cl₃-Indikators umfasst folgende Schritte:
a) Bildung von Tetraphenylporphyrin durch Reaktion von Pyrrol mit Benzaldehyd in siedender Propionsäure: Das TPP-P(V)Cl₃ wird zunächst in einer zweistufigen Synthese hergestellt. Hierzu reagiert Pyrrol mit Benzaldehyd in siedender Propionsäure in einer zweistündigen Reaktion (nach Adler et al. (1967) A Simplified Synthesis for meso-Tetraphenylporphine. J. Org. Chem. 32 (2): 476) unter Bildung von meso-Tetraphenylporphyrin (TPP; Fig. 2). Das TPP kristallisiert mit ca. 20%iger Ausbeute beim Abkühlen aus. Nach Abfiltrieren, Waschen mit Methanol und Trocknen bei ca. 120 °C kann das saubere Rohprodukt phosphoryliert werden.
b) Bildung von TPP-P(V)Cl₃ aus Tetraphenylporphyrin durch Phosphorylierung des Tetraphenylporphyrins durch Reaktion mit Phosphortrichlorid und Phosphorylchlorid in siedendem Pyridin: Hierzu reagiert TPP mit einem Überschuss eines 1:1 Gemisches aus Phosphortrichlorid und Phosphorylchlorid in siedendem Pyridin (Fig. 3).
c) Entfernen des Pyridins: Das Pyridin wird bevorzugt abdestilliert.
d) Reinigung des TPP-P(V)Cl₃: Diese erfolgt bevorzugt säulenchromatographisch über Aluminumoxid.
e) Aktivierung des TPP-P(V)Cl₃ durch kovalente Bindung an Kieselgel durch die Reaktion eines der Chlor-Liganden des TPP-P(V)Cl₃ mit einer Silanolgruppe des Kieselgels: Für die Herstellung des aktiven Indikators wird das mit grüner Farbe in Dichlormethan (DCM) gelöste TPP-P(V)Cl₃ mit Kieselgel unter Feuchtigkeitsausschluss vermischt und das Lösungsmittel im Rotationsverdampfer langsam abgedampft. Im Anschluss reagiert ein Chlor-Ligand des Phosphorporphyrins mit einer Silanolgruppe des oberflächenreichen Kieselgels beziehungsweise mit einer Silanolgruppe an der Oberfläche von Nanopartikeln, bevorzugt bei einer Temperatur zwischen 80 und 140 °C, bevorzugt bei 120 °C, im Trockenschrank für 8 bis 30 Stunden (Fig. 4).

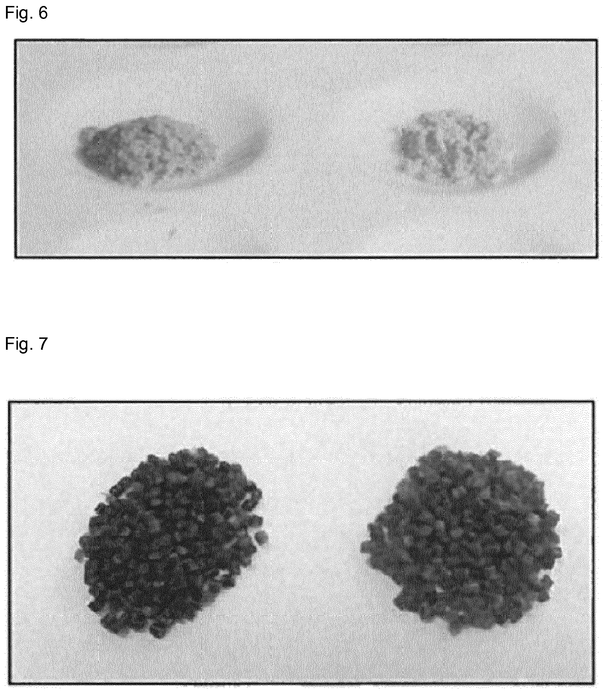

Durch diese spezielle Reaktion ist der zweite Chlor-Ligand des TPP-P(V)Cl₃ aktiviert (Fig. 5), so dass es sehr empfindlich mit Spuren von Ammoniak und/oder Aminen reagieren kann. Durch die Aktivierung kann nicht nur mit Ammoniak und/oder Aminen aber auch mit Wasser eine Reaktion erfolgen, welche mit einem visuell wahrnehmbaren Farbwechsel von grün nach rot verbunden ist (Fig. 6).

Bei der Aktivierung des Phosphorporphyrins ist es sehr wahrscheinlich, dass durch sterische Hinderung nur ein Chlor-Ligand zunächst mit einer Silanolgruppe des Kieselgels in einer Festphasenreaktion reagiert. Dadurch wird der zweite Chlor-Ligand extrem aktiviert, und eine überraschend irreversible Reaktion sowohl mit Wasser als auch mit Ammoniak und/oder Aminen kann erfolgen.

In der US7772215 "Water detection composition and water detection indicator" wird ebenfalls zunächst ein Phosphorporphyrin mit zwei Axialen Chlor-Liganden hergestellt. In den meisten Beispielen werden durch eine weitere Synthese die Chlor-Liganden gegen Hydroxy-Liganden ausgetauscht bevor der neue Phosphor-Komplex bei Anwesenheit von Calciumchlorid an Kieselgel adsorbiert wird. In einem Beispiel wird direkt das Phosphorporphyrin mit axialen Chlor-Liganden auf diese Weise an Kieselgel adsorbiert. Dann wird das Kieselgel bei 100°C getrocknet und kann Feuchtigkeit detektieren (wasserfreies Kieselgel ist grün; feuchtes Kieselgel ist rot bis rotbraun bzw. pink). Dieser Prozess kann hier durch Trocknung des Kieselgels durch Erwärmen umgekehrt werden und ist somit reversibel. Bei der Absorption des Phosphorporphyrin-Komplexes handelt es sich um elektrostatische Wechselwirkungen zu den Silanolgruppen des Kieselgels. Hierbei kommt es zu keiner kovalenten Bindung mit den Silanolgruppen.

Im Gegensatz zur US7772215 reagieren die Silanolgruppen des Kieselgels bei der vorliegenden Erfindung mit dem Phosphor-Zentralatom unter Ausbildung einer kovalenten Bindung, da hier der Phosphorkomplex mit den Chlor-Liganden mit den Silanolgruppen des Kieselgels bei erhöhter Temperatur, bevorzugt zwischen 80 und 140 °C, und bevorzugt für 8 bis 30 Stunden, zur chemischen Reaktion gebracht wird. Obwohl der Kieselgel-Porphyrin-Komplex der vorliegenden Erfindung ebenfalls mit Wasser einen Farbwechsel von grün nach rot zeigt, findet bei der Trocknung (z.B. durch Erhitzen) keine Farbrückveränderung von rot nach grün statt. Der entscheidende Unterschied zur US7772215 besteht also darin, dass der wasserbedingte Farbwechsel des erfindungsgemäßen Indikators irreversibel ist. Die Begründung hierfür liegt darin, dass bei der vorliegenden Erfindung das Phosphorporphyrin kovalent an einer Silanolgruppe des Kieselgels gebunden ist, und nur der zweite noch vorhandene Chlor-Ligand mit Wasser reagiert.

Für einen zuverlässigen Nachweis von Ammoniak und/oder Aminen mit dem erfindungsgemäßen Dosimeter-Material muss die Nebenreaktion des Indikators mit Wasser, die ebenfalls einen Farbwechsel von grün nach rot verursacht und äußerst empfindlich auf Feuchtigkeitsspuren in der Gasphase ist, unterbunden werden. Nach der Herstellung des Indikators, z.B. des TPP-P(V)Cl₃-Indikators, muss dieser also vor Feuchtigkeitsspuren geschützt werden. Gleichzeitig darf die Diffusion von Ammoniak und/oder Amine und der Kontakt mit dem aktiven Indikator nicht verhindert werden. Durch Einbettung des aktiven Indikators in eine semipermeable Matrix, d.h. eine für Ammoniak und/oder Amine permeable und für Wasser undurchlässige Immobilisierungsmatrix, wird die Querempfindlichkeit zu Wasser ausgeschlossen. Entscheidend bei der vorliegenden Erfindung ist somit die Einbettung des feuchtigkeitsempfindlichen Indikators in eine Immobilisierungsmatrix, bevorzugt in eine Polymermatrix. Das aminpermeable Polymer fungiert hierbei nicht nur als Träger/Immobilisierungsmatrix für den Indikator, sondern verhindert auch einen durch Feuchtigkeit bedingten Farbwechsel des Indikators und ist somit eine essentielle Komponente in der Funktion des erfindungsgemäßen Dosimeter-Materials.

Als Immobilisierungsmatrix sind Polymere geeignet, die keine Permeabilität für Wasser (inkl. Wasserdampf) aufzeigen, aber permeabel für Ammoniak und/oder Amine sind. Insbesondere kommt Low-Density Polyethylen (LDPE; Dichte zwischen 0,910 und 0,940 g/cm³) in Frage. Polymere wie Polystyrol (PS) sind ebenfalls geeignet als Immobilisierungsmatrix. Ferner ist eine Polymermischung denkbar, solange ein solches Mehrkomponentensystemen die für eine erfindungsgemäße Immobilisierungsmatrix erforderlichen physikalischen Eigenschaften bezüglich Gasdiffusion und Wasseraufnahme aufweist.

Für die Herstellung des auf PS-basierenden Dosimeter-Materials wird der Indikator in eine hoch viskose Lösung aus Polystyrol in Toluol eingerührt, und dann zu dünnen Schichten von ca. 1-2 mm Stärke ausgegossen. Nach dem Abdampfen des Toluols entsteht eine hoch aktive Folie. Ein Nachteil dieses Herstellungsprozesses ist allerdings der eventuelle verfahrensbedingte Lösungsmittelrest in der Folie, der die darin verpackten Lebensmittel belasten könnte. Die potentielle toxische Belastung kann mittels einer durch thermische Extrusion hergestellten Folie vermieden werden.

Eine bevorzugte Alternative zu diesem Herstellungsprozess ist somit eine thermische Vermischung des Indikators mit dem stark hydrophoben Polymer LDPE, welches eine gute Permeabilität für Ammoniak und Amine aufweist. LDPE ist auch aus dem Stand der Technik bekannt für eine äußerst geringe Wasseraufnahme, gleichzeitig besitzt es eine hohe Permeabilität gegenüber Stickstoff, Sauerstoff, Kohlendioxid, sowie vielen Geruchs- und Aromastoffen. Das grüne Indikatorpulver wird thermisch über Extrusion im Polymer verteilt. LDPE besitzt den Vorteil einer niedrigen Verarbeitungstemperatur von 160 - 220 °C. In diesem Prozess entsteht ein grünes Granulat. Die Anwesenheit von Aminen führt zu einen Farbwechsel des Granulats von grün nach rot (Fig. 7). Das grüne Granulat kann im Anschluss zu einer Folie verarbeitet werden, bei der in Anwesenheit von Aminen ebenfalls ein Farbwechsel von grün nach rot erfolgt.

Der Indikator kann der Immobilisierungsmatrix in beliebigen Mengen zugesetzt werden. Vorzugsweise wird der Indikator der Immobilisierungsmatrix in einer Menge zugesetzt, die gerade ausreicht, um der Immobilisierungsmatrix eine genügende Färbung zu verleihen, um mit bloßem Auge erkennbar zu sein. Gemäß einer besonders bevorzugten Ausführungsform wird der Indikator der Immobilisierungsmatrix in einer Menge von 0,1 bis 5,0 % (w/w), bezogen auf die Gesamtmenge der Immobilisierungsmatrix, zugesetzt.

Durch lichtstreuende Additive in unterschiedlichen Verhältnissen zum Phosphorporphyrin ist eine Empfindlichkeitssteigerung durch Erhöhung des optischen Kontrastes möglich. Insbesondere Titanoxid, welches als Weißpigment aus der Farbindustrie bekannt ist, führt beim Indikator zu einer besseren Sichtbarkeit des visuell und kolorimetrisch detektierbaren Farbumschlags von grün nach rot.

Die Problematik der Wasserquerempfindlichkeit des Indikators wird durch die Einbettung in der Immobilisierungsmatrix gelöst: Selbst nach mehreren Wochen Immersion in Wasser ist kein feuchtigkeitsbedingter Farbwechsel bei LDPE-Dosimeter-Folien zu beobachten.

Das erfindungsgemäße Dosimeter-Material weist Selektivität für Ammoniak und/oder Amine auf. Es zeigt ein besonders gutes Ansprechverhalten bei Aminen mit einer molaren Masse unter 150 g/mol. Beispiele für Amine im Sinne der vorliegenden Erfindung sind Diethylamin, Trimethylamin, Triethylamin, Ethanolamin, Hexylamin, Cadaverin und Putrescin. Bei Querempfindlichkeitstests wurde kein Farbwechsel des Dosimeter-Materials mit Thiolen, Aminosäuren, Alkoholen, Aldehyden oder Ketonen beobachtet (s. Beispiel 6).

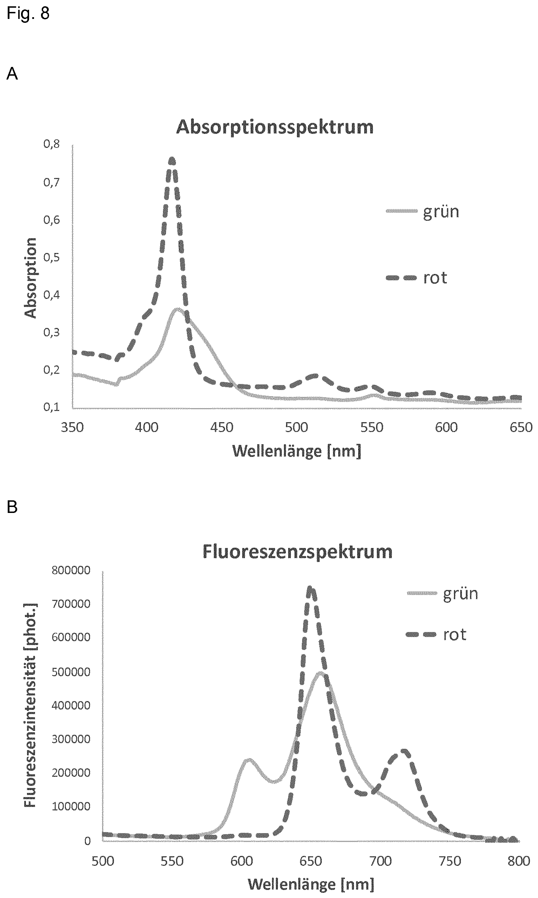

Das erfindungsgemäße Dosimeter-Material weist hohe Sensitivität für Ammoniak und/oder Amine auf. Ein kolorimetrisch detektierbarer Farbumschlag erfolgt bei einer Sensorfläche von einem Quadratzentimeter und einer Folienstärke von 100 µm im Bereich von minimal 20 nmol. Durch messtechnische Auswertung, insbesondere von Fluoreszenzeigenschaften, lässt sich diese Empfindlichkeit mindestens um den Faktor 100 verbessern. Die deutliche Änderung der Absorptions- und Fluoreszenzspektren des Dosimeter-Materials nach Reaktion mit Ammoniak und/oder Aminen ist in Fig. 8 dargestellt.

Der qualitative und/oder quantitative Nachweis von Ammoniak und/oder Aminen, insbesondere in einem Gasgemisch, kann durch ein Verfahren erfolgen, welches folgende Schritte umfasst:
a) Bereitstellen eines erfindungsgemäßen Dosimeter-Materials;
b) Wechselwirken des Ammoniaks und/oder der Aminen mit dem Dosimeter-Material;
c) Messen einer Fluoreszenzeigenschaft und/oder Absorptionseigenschaft zumindest eines Abschnitts des Dosimeter-Materials.

Die Quantifizierung über die Absorptionseigenschaften des Indikators kann über den Bereich zwischen 490 und 530 nm oder auch über den Bereich 400 bis 450 nm erfolgen (Fig. 8 A).

Die Fluoreszenz ist, im Gegensatz zur Absorption, hintergrundfrei, und Änderungen im Fluoreszenzspektrum sind wesentlich empfindlicher zu messen. Im Fluoreszenzspektrum kommt es nach Anregung im Wellenlängenbereich zwischen 400 und 450 nm oder durch Multiphotonenanregung im Bereich 700 bis 800 nm zu einer signifikanten Änderung der Maxima bei 600 nm, 650 nm und 720 nm (Fig. 8 B). Zur genauen Quantifizierung kann die Bestimmung des Verhältnisses zweier dieser Maxima herangezogen werden.

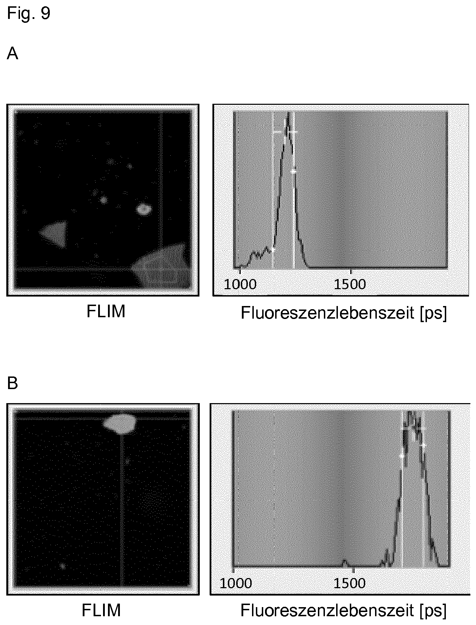

Die Reaktion des Dosimeter-Materials mit Aminen führt zudem zu einer deutlichen Änderung der Fluoreszenzlebenszeit. Fig. 9 zeigt auf der linken Seite Aufnahmen mit Farbkodierung für die Fluoreszenzlebenszeit des Dosimeter-Materials (A: grün; B: rot). Die Fluoreszenzlebenszeitmessungen wurden am Multiphotonenmikroskop mit zeitkorrelierter Einzelphotonen-Detektion durchgeführt. Der schwarze Hintergrund besteht aus der Immobilisierungsmatrix, der eingebettete Indikator erscheint als Partikel mit einer Größe von 20 bis 90 nm. Auf der rechten Seite ist die quantitative Auswertung der Farbkodierung dargestellt. Parallel zum Farbwechsel von grün nach rot steigt die Fluoreszenzlebenszeit nach Reaktion des Indikators mit Aminen signifikant von 1100 - 1300 ps auf 1600 - 1800 ps.

Als biogene Amine werden Decarboxylierungsprodukte von Aminosäuren bezeichnet. Biogene Amine sind in geringen Konzentrationen in Lebensmitteln ubiquitär vorhanden. Ab bestimmten Konzentrationen können biogene Amine die menschliche Gesundheit negativ beeinträchtigen, sie verursachen pharmakologische, physiologische und toxische Wirkungen. Ihre Mengen nehmen häufig als Folge der Verwendung von Rohstoffen minderer Qualität, während kontrollierter oder spontaner mikrobieller Fermentation oder im Laufe des Lebensmittelverderbs zu. Besonders betroffen sind Lebensmittel wie Fisch, Fleisch und Wurst, Käse, Wein, Bier, Sauerkraut, Sojasoße und Hefeextrakt. Biogene Amine eignen sich aus diesem Grund besonders gut als chemische Indikatoren für die hygienische Qualität und Frische ausgewählter Lebensmittel, welche zu einem gewissen Grad mit einer Fermentation oder Abbau assoziiert sind.

Das erfindungsgemäße Dosimeter-Material bietet gegenüber dem Stand der Technik den großen Vorteil einer direkten Detektion potentiell gesundheitsschädlicher Amine. Das Dosimeter-Material kann direkt freigesetztes Ammoniak und/oder Amine durch Änderung von Farbe, Absorptions- und Fluoreszenzeigenschaften anzeigen. Diese Änderungen korrelieren mit einer quantifizierbaren Änderung (Zunahme) der Ammoniak- und/oder Aminkonzentration, so dass der Zustand der zu untersuchenden Proben, insbesondere von biologischen Testmaterialen, kontinuierlich prospektiv überwacht werden kann.

Im Prinzip können mit dem erfindungsgemäßen Dosimeter-Material alle Fragestellungen verfolgt werden, bei denen Ammoniak und/oder Amine im Sinne von Verderb, Alterung oder Reifung freigesetzt werden. Dies trifft im Themenfeld Lebensmittel auf alle Produkte tierischen Ursprungs zu, da nach der Schlachtung oder der Produktverarbeitung nachhaltige Abbauprozesse einsetzen, die ab einem bestimmten Zeitpunkt, den Verzehr des Lebensmittels beeinflussen. In anderen Fällen zeigt eine gesteigerte Aminbildung auch einen Reifungsprozess an, der positiv bewertet werden kann (z.B. bei der Käsereifung oder Matjesproduktion). In letzteren Fällen kann das Dosimeter-Material auch als Reifeindikator eingesetzt werden.

Lebensmittelverpackungen sind in der Regel nicht permeabel für Geruchs- und Aromastoffe. Das Dosimeter-Material kann auf der Innenseite der Verpackung aufgebracht werden, so dass der Farbwechsel nur von den Aminen von dem jeweiligen Material in der Verpackung stammt und nicht von evtl. Aminen in der Atmosphäre. Hierfür sollte die Verpackungsfolie für Amine undurchlässig sein, und der Indikator kann zum Verpackungsgut mit einer Amin-durchlässigen Folie abgegrenzt werden. Es könnten auch Sandwich-Folien verwendet werden.

Der bereits bei Spuren von Ammoniak und/oder Aminen nachweisbare Farbwechsel von grün nach rot eignet sich besonders gut für den Einsatz im Bereich intelligenter Lebensmittelverpackungen. In einer intelligenten Lebensmittelverpackung kann durch das Dosimeter-Material, vom Transport über Händler bis zum Endverbraucher, die Haltbarkeit von Lebensmitteln direkt abgelesen werden (grün = frisch; rot = nicht mehr frisch, erste Anzeichen von Verderb). Dieser Ansatz besticht durch den einfachen, mobilen Nachweis für Ammoniak und/oder Amine ohne eine aufwändige Laboranalytik.

Darüber hinaus ist eine schnelle sensitive automatisierte Beurteilung der Haltbarkeit von Lebensmitteln möglich. Durch die Detektion von Fluoreszenz bzw. Absorption kann das Dosimeter-Material sehr vielfältig eingesetzt werden.

Der Nachweis von Ammoniak und/oder Aminen kann also mit dem Dosimeter-Material sowohl einfach und leicht verständlich für einen Endverbraucher erfolgen als auch quantifizierbar für den industriellen Anwender sein. Auf dem Markt der intelligenten Verpackungen gibt es zurzeit kein vergleichbares Produkt mit solchen vielfältigen Einsatzmöglichkeiten:

### 1. Dosimeter für den Endverbraucher

Die Verpackung ist als Trägerin der Lebensmittelkennzeichnung eine wesentliche Informationsquelle für Verbraucher. Sie hat daher einen erheblichen Einfluss auf die Kaufentscheidung. In der Regel kann der Verbraucher die Frische eines in Kunststofffolie verpackten Lebensmittels im Supermarkt schlecht einschätzen, da die sensorische Analyse anhand von Olfaktorik, Haptik und Optik nur eingeschränkt möglich sind. Für den Endverbraucher, der beim Einkauf schnell eine Aussage über die Haltbarkeit des gewünschten Lebensmittels benötigt, sind die meisten der zurzeit auf dem Markt befindlichen oder der neu entwickelten Beurteilungsverfahren nicht geeignet, da sie zu kompliziert und zu teuer sind, oder gesundheitsschädliche Farbstoffe enthalten. Ein Haltbarkeitsdosimeter muss sehr kostengünstig herzustellen sein und muss außerdem eine Signalwirkung haben, die leicht zu erkennen ist (am besten wie eine Ampel: grün => guter Zustand; rot => das Lebensmittel ist verändert). Der Endverbraucher wird beim Kauf von z.B. abgepacktem Fisch oder Fleisch direkt durch den Farbwechsel des Dosimeter-Materials in der Verpackung über die Frische des Produktes informiert.

### 2. Dosimeter für lebensmittelverarbeitende Betriebe und den Lebensmitteleinzelhandel

Für lebensmittelverarbeitende Betriebe ist das Dosimeter-Material ebenfalls interessant, um die Haltbarkeit der verpackten Lebensmittel zu überprüfen. Das Dosimeter-Material muss in diesem Fall für den Endverbraucher nicht unbedingt sichtbar sein. Mit dem Dosimeter-Material könnte eine kontinuierliche Kontrolle während des gesamten Produktions- und Transportprozesses sichergestellt werden, da der Farbwechsel automatisiert analysiert und quantifiziert werden kann. Mit einer automatisierten Onlinekontrolle könnte die bisher übliche stichprobenartige Prüfung durch eine schnelle Überprüfung jeder einzelnen Packung ersetzt werden. Der Produzent von Lebensmitteln könnte zudem mit einem solchen Frischeindikator für die Sicherheit seines Produktes werben, da selbst durch das branchenübliche Umpacken der Ware das Dosimeter-Material nicht manipuliert werden kann.

Beim Lebensmitteleinzelhandel könnte zudem, beispielsweise durch Messung der Fluoreszenzintensität mit einem entsprechenden Messgerät, die (tagesaktuelle) Frische quantitativ beurteilt werden. Dies könnte auch dazu beitragen, dass weniger Lebensmittel zu Zeitpunkten entsorgt werden, wo ihre bedenkliche Haltbarkeit noch nicht erreicht ist.

### 3. Andere Anwendungen

Das Dosimeter-Material kann ferner in der Medizinanalytik zum Einsatz kommen. Sollte ein Porphyrin-basierter Dosimeter auf die gewünschte Empfindlichkeit eingestellt und sein zeitliches Ansprechverhalten modifiziert werden, kann es auch auf dem medizinischen Sektor, beispielsweise in der klinischen Diagnostik, genutzt werden. Bei der Atemgasanalyse von Nierenversagen spielen z.B. die Amine Di- und Trimethylamin eine wichtige Rolle. Hierfür ist eine sehr sensitive Detektion im ppm-Bereich notwendig. Das Dosimeter-Material kann hier als Teststreifen genutzt oder in einem Atemluftbeutel integriert werden, um die Reaktionsmöglichkeit der ausgeatmeten Amine mit der Folie zu verbessern. Auch weitere medizinische Indikationen, die in Zusammenhang mit der Bildung von Aminen einhergehen, z.B. in der Zahnmedizin, sind denkbar. Durch die gesteigerte Sensibilität und die Möglichkeit der quantitativen Auswertung ist ebenfalls eine Anwendung bei Wundheilverbänden ("smart bandage") denkbar.

Auch auf dem Gebiet der Umweltanalytik kann ein solcher Sensor eingesetzt werden, z.B. für den Gewässer- und Bodenschutz.

Das erfindungsgemäße Dosimeter-Material und das entsprechende Verfahren zum Nachweis von Ammoniak und/oder Aminen bieten zahlreiche Vorteile:
1. Es werden direkt die beim Verderb (bzw. Reifung) entstehenden Stoffe, nämlich Ammoniak und/oder Amine, detektiert, ohne einen Umweg beispielsweise durch eine pH-Bestimmung.
2. Die Detektion findet bevorzugt in der Gasphase statt, das Dosimeter-Material muss also nicht zwangsläufig mit dem Lebensmittel in Berührung kommen, was den Einsatz bei einer breiten Vielfalt von Verpackungsarten ermöglicht.
3. Die Detektion von Ammoniak und/oder Aminen ist sehr sensitiv und selektiv, es können sogar Spuren schwerflüchtiger biogener Amine wie z.B. Cadaverin und Putrescin detektiert werden.
4. Die Änderungen in Absorption und Fluoreszenz des Indikators sind weitaus ausgeprägter als bei anderen bekannten Aminindikatoren. Vor allem bei der Fluoreszenz kommt es zur Ausbildung von sehr charakteristischen Fluoreszenzmaxima. Aber auch der mit dem Auge erkennbare Farbwechsel von grün nach rot ist gut sichtbar und interpretierbar.
5. Der Indikator reagiert in einer irreversiblen Reaktion mit Ammoniak und/oder Aminen, sodass ein "Zurückfärben" nicht möglich ist. Dadurch sind etwaige Manipulationen wie Umverpacken auf dem Weg zum Endverbraucher erschwert.
6. Das Dosimeter-Material zeichnet sich im Vergleich zu anderen bekannten Indikatoren für Ammoniak und/oder Aminen durch seine gesundheitliche Unbedenklichkeit aus, weder toxische noch kanzerogene Wirkungen sind bekannt. Die einzelnen Komponenten, Porphyrin (z.B. auch TPP und TPP-P(V)Cl₃), Kieselgel und Polymere gelten als gesundheitlich unbedenklich.
7. Das Dosimeter-Material kann kostengünstig hergestellt werden und würde damit ein Wegwerf-Dosimeter ermöglichen, besonders interessant als Haltbarkeitsanzeige bei Lebensmittelverpackungen.

Die Erfindung wird anhand der folgenden Beispiele näher beschrieben.

### Beispiel 1: Synthese von meso-Tetraphenylporphyrin (TPP)

Es werden 100 g Benzaldehyd in 1,5 l Propionsäure vorgelegt und zum Sieden gebracht. Nach vorsichtigem Zutropfen von 63 g Pyrrol wird unter Rückflusskühlung für weitere 2 h erhitzt. Nach Abkühlen und Auskristallisieren des Porphyrins, wird die Suspension filtriert. Der violette Filterkuchen wird erst mit Propionsäure und im Anschluss mit Methanol gewaschen. Dann wird bei ca. 120 °C bis zur Gewichtskonstanz getrocknet. Ausbeute: 28,3 g (19,6 % d. Th.)

### Beispiel 2: Synthese von Dichloro-phosphor-(V)-tetraphenylporphyrin-chlorid

8 g TPP werden in 200 ml über Molsieb getrocknetem Pyridin unter Argon als Schutzgas vorgelegt. Dazu wird vorsichtig 40 ml eines 1:1 Gemisches aus Phosphortrichlorid und Phosphorylchlorid getropft und die dunkelrote Lösung 4 h unter Rückfluss zum Sieden erhitzt. Nach beendeter Reaktion wird die nun dunkelgrüne Lösung am Rotationsverdampfer bis zur Trockene eingeengt. Der Rückstand wird in wenig trockenem DCM aufgenommen und zunächst mit Hexan / DCM 1:2 über Aluminiumoxid chromatographisch gereinigt. Das Produkt lässt sich nach Abtrennen der Verunreinigungen mit DCM, versetzt mit ca. 1 % Ethanol von der Säule eluieren. Nach Abdampfen des Lösungsmittels am Rotationsverdampfer liegt nun das reine Phosphorporphyrin vor. Ausbeute: 7,9 g (64,7 % d. Th.).

### Beispiel 3: Herstellung des TPP-P(V)Cl₃-Indikators

Es werden 20 g Kieselgel 60 (0,040 - 0,063 mm, zur Säulenchromatographie) in einem Achatmörser so gut wie möglich pulverisiert. Das Kieselgel wird dann nach 24 h Trocknen bei 120 °C in eine Lösung von 200 mg TPP-P(V)Cl₃ in 60 ml getrocknetem DCM eingerührt. Nach Abdestillieren des Lösemittels am Rotationsverdampfer wird das grüne Indikatorpulver bei 120 °C für 24 h im Trockenschrank aktiviert. Die Abkühlung und Lagerung des Indikators erfolgt unter Feuchtigkeitsausschluss im Exsikkator.

### Beispiel 4: Herstellung des Dosimeter-Materials

In einem Prism Laborextruder wird aus einer Mischung von 910 g LDPE und 90 g Indikatorpulver ein Granulat hergestellt. Der Temperaturbereich für die Extrusion liegt zwischen 140 und 160 °C. Die Drehzahl der Doppelschnecke ist auf 250 upm eingestellt, so dass sich eine Verweilzeit von ca. 30 s ergibt. Der aus dem Extruder austretende heiße Kunststoffstrang wird in einem Wasserbad abgekühlt und zu Granulat zerkleinert. Im nächsten Schritt wird das grüne Granulat in einem Collin Flachfolienextruder 75D zu Folien weiter verarbeitet. Die Verarbeitungstemperatur liegt bei einer Verweilzeit von ca. 3 min zwischen 160 und 185 °C. Die aus der Düse austretende Folie wird über Walzen gekühlt und auf eine Stärke von 100 - 250 µm gebracht. Durch eine Schneckendrehzahl von 60 - 100 upm wird ein Druck von 150 - 200 bar im Extruder erzeugt. Sollte die Indikatorkonzentration für die Folie zu hoch sein, ist im zweiten Schritt bei der Folienherstellung durch Zusatz von reinem LDPE eine Verdünnung möglich.

### Beispiel 5: Reaktion des Dosimeter-Materials mit Ammoniak, Aminen und Wasser

Um das Ansprechverhalten des Dosimeter-Materials zu untersuchen, wurden Ammoniak und einige Amine sowohl mit dem Indikatorpulver als auch mit der Dosimeter-Folie getestet. Hierzu wurden etwa 10 mg des Pulvers oder ca. 1 cm² der Folie in ein 10 ml Probeglas mit Septum gegeben und mit einer Hamilton-Spritze 5 - 100 µl aus der Gasphase über dem jeweiligen Amin in das Reaktionsgefäß gespritzt. Der einzige Unterschied zwischen Folie und Pulver ist die Reaktionszeit der Farbreaktion. Schlägt bei dem Pulver die Farbe in der Regel sofort nach Gaszugabe um, ist bei der Folie der Prozess durch Diffusion gehindert und kann einige Zeit (bis zu einigen Stunden) dauern. Vorteil der Folie ist aber, dass eine Querempfindlichkeit zu Wasserdampf ausgeschlossen ist. Die Folie kann einige Tage im Wasser lagern, ohne dass sie ihre Farbe verändert. Tabelle 1 zeigt Siedepunkt und Dampfdruck von ausgewählten Aminen sowie die Farbreaktion dieser Amine mit dem Indikator:
+++ Farbreaktion nach Zugabe von geringem Gasphasenvolumen (Amine mit hohem Dampfdruck: > 100 hPa bei 20°C);
++ Farbreaktion nach Zugabe von mittlerem Gasphasenvolumen (Amine mit mittlerem Dampfdruck: 1-100 hPa bei 20°C);
+ Farbreaktion nach Zugabe von großem Gasphasenvolumen (Amine mit niedrigem Dampfdruck: < 1 hPa bei 20°C);
- keine Farbreaktion.

| **Amin** | **Siedepunkt bei 20°C [°C]** | **Dampfdruck bei 20 °C [hPa]** | **Farbreaktion** |
|---|---|---|---|
| Ammoniaklösung 25% | 37,7 | 483 | +++ |
| Diethylamin | 56 | 253 | +++ |
| Trimethylamin 31-23 wt% in Ethanol | ca. 31 | | +++ |
| Triethylamin | 90 | 69 | ++ |
| Ethanolamin | 171 | 0,5 | ++ |
| Hexylamin | 130 - 132 | 10,6 | ++ |
| 1,6-Diaminohexan | 199 - 204 | 0,25 | + |
| Cadaverin (1,5-Diaminopentan) | 178 - 180 | | + |
| Puterecin (1,4-Diaminobutan) | 158 - 160 | | + |
| Histamin | 167 (bei 1,1 hPa) | | + |
| Triethanolamin | 360 | <0,01 | - |

Ebenfalls zeigten Vorversuche mit der Dosimeter-Folie mit altem Fisch bzw. Fleisch nach einiger Zeit eine positive Farbreaktion.

### Beispiel 6: Querempfindlichkeit zu anderen Stoffen, die beim Verderb von Lebensmitteln auftreten können

Es wurden unterschiedliche niedermolekulare Verbindungen in hoher Konzentration mehrere Tage sowohl mit dem hochempfindlichen Indikatorpulver als auch mit der Dosimeter-Folie in Kontakt gebracht. Bei diesen Querempfindlichkeitstests wurde kein Farbwechsel mit Schwefelwasserstoff, Thiolen, Aminosäuren, Alkoholen, Aldehyden oder Ketonen beobachtet.

### Beispiel 7: Vereinfachte Betrachtungen zur Empfindlichkeit des Dosimeter-Materials:

Im Folgenden soll abgeschätzt werden welche Stoffmenge eines Amins nötig ist um das Dosimeter-Material von grün nach rot wechseln zu lassen. Als Modellbetrachtung wird von einem Dosimeter-Folienspot mit einer Fläche von 1 cm² ausgegangen. Die Folienstärke beträgt 250 µm. Bei einer Dichte des LDPE von ca. 1 g/cm³ hat der Dosimeter-Folienspot eine Masse von 25 mg. Die hierin enthaltene Indikatorkomponente beträgt mit 3 % Anteil 0,75 mg Pulver. Der Indikator enthält 1 % der aktiven Porphyrinkomponente, also befinden sich im Sensorspot 7,5 µg TPP-P(V)Cl₃. Da die molare Masse des Porphyrins 750 g/mol beträgt, lässt sich eine Stoffmenge von 10 nmol errechnen. Wird davon ausgegangen, dass das Porphyrin für die Farbreaktion 1:1 mit Aminen reagiert, muss der Dosimeter-Folienspot ca. 10 nmol Amin aus der Gasphase aufnehmen. Wird nun beispielsweise 1,6-Diaminohexan mit einem Dampfdruck von 0,25 hPa bei 20 °C als hochsiedende Modellverbindung für Amine betrachtet und wird davon ausgegangen, dass die Lebensmittelverpackung ein Gasvolmen von 0,25 l enthält berechnet sich aus der idealen Gasgleichung, dass sich von diesem hochsiedendem Amin ca. 2,5 µmol in der Gasphase befinden: $N = \frac{p * V}{R * T} = {25 Pa * 0,25 l}_{¯} = 2,5 μmol$ 8314,5 [Pa l mol⁻¹ K⁻¹]* 293,15 K

Aus diesen Betrachtungen ergibt sich, dass in der Gasphase ausreichend Amin vorhanden ist, um einen Farbwechsel des Dosimeter-Folienspot zu induzieren.

## Patentansprüche

1. Indikator, der in Anwesenheit von Ammoniak und/oder Aminen einen irreversiblen Farbwechsel erfährt, **dadurch gekennzeichnet, dass** der Indikator ein durch kovalente Bindung an eine Silanolgruppe aktiviertes Phosphorporphyrin umfasst, wobei eine Aktivierung von Phosphorporphyrin mit der Formel Porphyrin-P(V)X₃, wobei X Cl oder Br ist, durch eine kovalente Bindung durch die Reaktion nur eines der Halogen-Liganden des Porphyrin-P(V)X₃ mit der Silanolgruppe erfolgt, wobei die Silanolgruppe Teil eines Stoffes ist, der eine Vielzahl von Silanolgruppen aufweist und eine hohe Oberfläche hat.

2. Indikator nach Anspruch 1, **dadurch gekennzeichnet, dass** der Indikator ein durch kovalente Bindung an eine Silanolgruppe von einem Kieselgel umfassenden Stoff aktiviertes Phosphorporphyrin umfasst, wobei der Stoff bevorzugt Kieselgel ist.

3. Indikator nach einem der Ansprüche 1 oder 2, **dadurch gekennzeichnet, dass** das Phosphorporphyrin Dichloro-phosphor-(V)-tetraphenylporphyrin-chlorid (TPP-P(V)Cl₃), Dibromo-phosphor-(V)-tetraphenylporphyrin-bromid (TPP-P(V)Br₃), Dichloro-phosphor-(V)-tetratolylporphyrin-chlorid (TTP-P(V)Cl₃), oder Dichloro-phosphor-(V)-2,3,7,8,12,13,17,18-octaethylporphyrin-chlorid (OEP-P(V)Cl₃) ist, bevorzugt Dichloro-phosphor-(V)-tetraphenylporphyrin-chlorid (TPP-P(V)Cl₃).

4. Indikator nach einem der Ansprüche 1 - 3, umfassend:

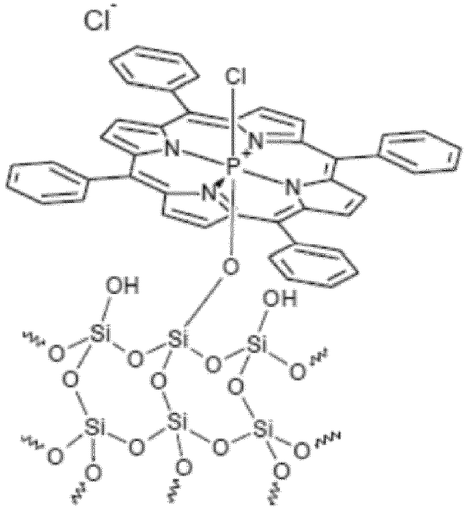

5. Dosimeter-Material für Ammoniak und/oder Amine, insbesondere in der Gasphase, umfassend einen Indikator nach einem der Ansprüche 1 bis 4 und eine für Ammoniak und/oder Amine permeable Immobilisierungsmatrix für den Indikator, wobei die Immobilisierungsmatrix wasserundurchlässig ist.

6. Dosimeter-Material nach Anspruch 5, **dadurch gekennzeichnet, dass** das Dosimeter-Material als Granulat und/oder Folie vorliegt, bevorzugt als Folie.

7. Dosimeter-Material nach einem der Ansprüche 5 oder 6, **dadurch gekennzeichnet, dass** die Immobilisierungsmatrix für Wasser undurchlässige und für Ammoniak und/oder Amine durchlässige Polymere aus der Gruppe umfassend Polystyrol und Low-Density Polyethylen und eine Kombination davon umfasst, bevorzugt Low-Density Polyethylen.

8. Verfahren zur Herstellung des Indikators nach einem der Ansprüche 1 bis 4, **dadurch gekennzeichnet, dass** eine Aktivierung des Porphyrin-P(V)X₃ durch eine kovalente Bindung durch die Reaktion nur eines der Halogen-Liganden des Porphyrin-P(V)X₃ mit der Silanolgruppe, bevorzugt von Kieselgel, erfolgt.

9. Verfahren nach Anspruch 8, **dadurch gekennzeichnet, dass** eine Aktivierung von Dichloro-phosphor-(V)-tetraphenylporphyrin-chlorid durch kovalente Bindung an Kieselgel durch die Reaktion eines der Chlor-Liganden des Dichloro-phosphor-(V)-tetraphenylporphyrin-chlorids mit einer Silanolgruppe des Kieselgels erfolgt.

10. Verfahren zur Herstellung des Dosimeter-Materials nach einem der Ansprüche 5 bis 7, umfassend Vermischen des Indikators, optional hergestellt nach einem der Ansprüche 8 oder 9, mit der Immobilisierungsmatrix, wobei das Verfahren unter Ausschluss von Wasser durchgeführt wird, und das Gemisch aus Indikator und Immobilisierungsmatrix bevorzugt als Granulat und/oder Folie hergestellt wird.

11. Verwendung des Dosimeter-Materials nach einem der Ansprüche 5 bis 7 für die Detektion von Ammoniak und/oder Aminen, wobei der Farbwechsel des Indikators bevorzugt visuell oder durch die Detektion von Fluoreszenz bzw. Absorption erfasst werden kann.

12. Verwendung nach Anspruch 11, wobei die Verwendung in der Qualitätskontrolle von Lebensmitteln oder in medizinischen Anwendungen wie der Atemgasanalyse oder bei Wundheilverbänden erfolgt, bevorzugt in der Qualitätskontrolle von Lebensmitteln.

## Claims

1. An indicator which in the presence of ammonia and/or amines undergoes an irreversible colour change, wherein the indicator comprises a phosphorus porphyrin activated by a covalent bond to a silanol group, wherein an activation of phosphorus porphyrin with the formula porphyrin-P(V)X₃, wherein X is Cl or Br, occurs through a covalent bond from the reaction of only one halogen-ligand of the porphyrin-P(V)X₃ with the silanol group, wherein the silanol group is part of a compound which comprises a plurality of silanol groups and a large surface area.

2. The indicator according to claim 1, wherein the indicator comprises a phosphorus porphyrin activated by a covalent bond to a silanol group of a compound comprising silica gel, wherein the compound preferably is silica gel.

3. The indicator according to claim 1 or 2, comprising a phosphorus porphyrin that is dichlorophosphorus tetraphenylporphine chloride (TPP-P(V)Cl₃) , dibromophosphorus tetraphenylporphine bromide (TPP-P(V)Br₃), dichlorophosphorus tetratolylporphyrine chloride (TTP-P(V)Cl₃), or dichlorophosphorus 2,3,7,8,12,13,17,18-octaethylporphyrine chloride (OEP-P(V)Cl₃), preferably dichlorophosphorus tetraphenylporphine chloride (TPP-P(V)Cl₃).

4. The indicator according to any of claims 1 - 3, comprising:

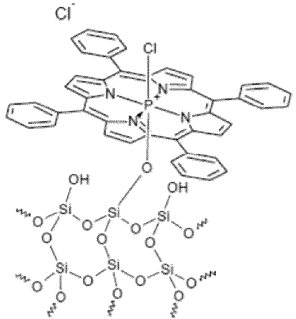

5. A dosimeter material for ammonia and/or amines, particularly in gaseous form, comprising an indicator according to any of claims 1 - 4 and an immobilisation matrix for the indicator permeable to ammonia and/or amines, wherein the immobilisation matrix is impermeable to water.

6. The dosimeter material according to claims 5, wherein the dosimeter material is present as granules and/or film, preferably as film.

7. The dosimeter material according to any of claims 5 or 6, wherein the immobilisation matrix comprises polymers impermeable to water and permeable to ammonia and/or amines selected from the group comprising polystyrene, low-density polyethylene, and a combination thereof, preferably low-density polyethylene.

8. A method for producing the indicator according to any of claims 1 - 4, wherein activation of porphyrin-P(V)X₃ occurs through a covalent bond from the reaction of only one halogen ligand of the porphyrin-P(V)X₃ with the silanol group, preferably of silica gel.

9. A method according to claim 8, wherein the activation of dichlorophosphorus tetraphenylporphine chloride occurs through a covalent bond to silica gel from the reaction of one of the chloride ligands of the dichlorophosphorus tetraphenylporphine chloride with a silanol group of silica gel.

10. A method for producing the dosimeter material according to any of claims 5 to 7, comprising mixing the indicator, optionally produced according to any of claims 8 or 9, with the immobilisation matrix, wherein the method is performed under exclusion of water, and the mixture of indicator and immobilisation matrix is preferably produced as granules and/or film.

11. A use of the dosimeter material according to any of claims 5 to 7 for the detection of ammonia and/or amines, wherein the colour change of the indicator can preferably be determined visually or by the detection of fluorescence or absorption thereof.

12. The use of claim 11, wherein the use occurs in quality control of food or in medical applications such as breath analysis or in wound dressings, preferably in quality control of food.

## Revendications

1. Indicateur qui subit une changement irréversible de couleur en présence d'ammoniac et/ou d'une amine, **caractérisé en ce que** cet indicateur comprend une phosphoro-porphyrine activée par liaison covalente à un groupe silanol, étant entendu que l'activation de la phosphoro-porphyrine, de formule porphyrine-P(V)X₃ ou X représente Cl ou Br, résulte de la formation d'une liaison covalente par réaction d'un seul des ligands halogéno de la porphyrine-P(V)X₃ avec le groupe silanol, et que ce groupe silanol fait partie d'une substance qui comporte un grand nombre de groupes silanol et présente une surface de grande étendue.

2. Indicateur conforme à la revendication 1, **caractérisé en ce que** cet indicateur comprend une phosphoro-porphyrine activée par liaison covalente à un groupe silanol d'une substance comprenant un gel de silice, étant entendu que, de préférence, cette substance est un gel de silice.

3. Indicateur conforme à l'une des revendications 1 et 2, **caractérisé en ce que** la phosphoro-porphyrine est du chlorure de dichloro-phosphoro-(V)-tétraphényl-porphyrine (TPP-P^{V}Cl₃), du bromure de dibromo-phosphoro-(V)-tétraphényl-porphyrine (TPP-P^{Y}Br₃), du chlorure de dichloro-phosphoro-(V)-tétratolyl-porphyrine (TTP-P^{V}Cl₃), ou du chlorure de dichloro-phosphoro-(V)-2,3,7,8,12,13,17,18-octaéthyl-porphyrine (OEP-P^{V}Cl₃), et de préférence, du chlorure de dichloro-phosphoro-(V)-tétraphényl-porphyrine (TPP-P^{V}Cl₃).

4. Indicateur conforme à l'une des revendications 1 à 3, comprenant

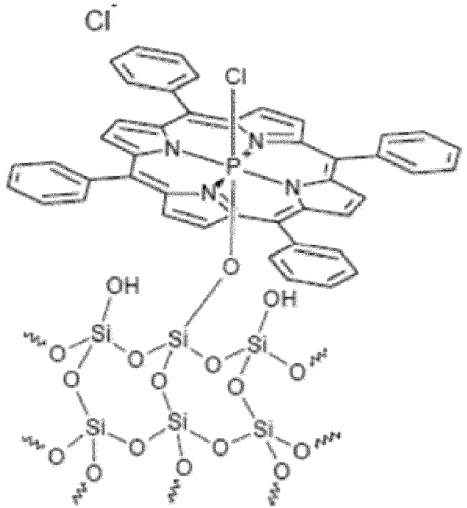

5. Matériau pour dosimétrie de l'ammoniac et/ou des amines, en particulier en phase gazeuse, comprenant un indicateur conforme à l'une des revendications 1 à 4, et pour immobiliser cet indicateur, une matrice perméable à l'ammoniac et/ou aux amines, laquelle matrice d'immobilisation est imperméable à l'eau.

6. Matériau pour dosimétrie conforme à la revendication 5, **caractérisé en ce que** ce matériau de dosimétrie se présente sous forme de granulat et/ou de feuille, et de préférence sous forme de feuille.

7. Matériau pour dosimétrie conforme à l'une des revendications 5 et 6, **caractérisé en ce que** la matrice d'immobilisation comprend des polymères imperméables à l'eau et perméables à l'ammoniac et/ou aux amines, choisis dans l'ensemble comprenant les polystyrène et polyéthylène basse densité et leurs combinaisons, mais de préférence, un polyéthylène basse densité.

8. Procédé de préparation d'un indicateur conforme à l'une des revendications 1 à 4, **caractérisé en ce que** l'activation de la porphyrine-P(V)X₃ résulte de la formation d'une liaison covalente par réaction d'un seul des ligands halogéno de la porphyrine-P(V)X₃ avec un groupe silanol, de préférence d'un gel de silice.

9. Procédé conforme à la revendication 8, **caractérisé en ce que** l'activation du chlorure de dichloro-phosphoro-(V)-tétraphényl-porphyrine résulte de la formation d'une liaison covalente avec un gel de silice, par réaction de l'un des ligands chloro du chlorure de dichloro-phosphoro-(V)-tétraphényl-porphyrine avec un groupe silanol du gel de silice.

10. Procédé de préparation d'un matériau de dosimétrie conforme à l'une des revendications 5 à 7, comportant le fait de mélanger l'indicateur, en option préparé selon l'une des revendications 8 et 9, avec la matrice d'immobilisation, étant entendu que le procédé est mis en œuvre à l'abri de l'eau et que le mélange de l'indicateur et de la matrice d'immobilisation est préparé, de préférence, sous forme de granulat et/ou de feuille.

11. Utilisation d'un matériau de dosimétrie, conforme à l'une des revendications 5 à 7, pour détecter de l'ammoniac et/ou des amines, dans laquelle on peut percevoir le changement de couleur de l'indicateur, de préférence, visuellement ou par détection d'une fluorescence ou d'une absorption.

12. Utilisation conforme à la revendication 11, laquelle utilisation intervient dans le contrôle de qualité des aliments, ou dans des applications médicales comme dans l'analyse de gaz expirés ou dans le pansage de blessures, et de préférence dans le contrôle de qualité des aliments.
